# EUROPEAN PATENT APPLICATION

(11) **EP 4 767 973 A1**
(43) Date of publication of application: **01.07.2026**
(21) Application number: 26150041.7
(22) Date of filing: 02.01.2026
(51) Int. Cl.: A61B 18/14, A61B 17/32

(54) **TECHNOLOGIES FOR USER FEEDBACK WITH MULTI-MODE ENERGY-BASED SURGICAL INSTRUMENTS**

(30) Priority: 31.12.2024 US 202463740970 P; 23.10.2025 US 202519367196
(71) Applicant: Cilag GmbH International, 6300 Zug (CH)
(72) Inventor: SHELTON, IV, Frederick E., Cincinnati, 45242 (US); HARRIS, Jason L., Cincinnati, 45242 (US); DRAGINOFF, Carl, Cincinnati, 45242 (US); KANE, Keith, Cincinnati, 45242 (US); ACKERMANN, Richard, Cincinnati, 45242 (US); ARONHALT, Jacqueline C., Cincinnati, 45242 (US)
(74) Representative: Carpmaels & Ransford LLP

(57) **Abstract**

A system includes an energy-based surgical instrument with a mode selector capable of selecting a latch mode and an automatic or non-latch mode. The surgical instrument may send a message signal indicative of the state of the mode selector to a surgical generator, which outputs an audio tone indicative of the state of the mode selector. The surgical instrument may generate feedback, such as haptic feedback, when energy delivery is nearly activated, and generate different feedback when the energy delivery is activated. The surgical instrument may measure tension applied at an end effector of the surgical instrument and generate haptic feedback based on the measured tension. Other embodiments are described and claimed.

## Description

### CROSS-REFERENCE TO RELATED APPLICATION

This application claims the benefit of and priority to U.S. Patent Application No. 63/740,970, entitled "TECHNOLOGIES FOR USER FEEDBACK WITH MULTI-MODE ENERGY-BASED SURGICAL INSTRUMENTS," which was filed on December 31, 2024, and which is incorporated herein by reference in its entirety.

### TECHNICAL FIELD

The present disclosure relates generally to energy-based surgical instruments and, more particularly, to harmonic and/or electrosurgical surgical instruments.

### BACKGROUND

Energy-based surgical instruments are finding increasingly widespread applications in surgical procedures by virtue of their unique performance characteristics. Depending upon specific device configurations and operational parameters, energy-based surgical instruments can provide both transection of tissue and hemostasis of the tissue by coagulation, which may reduce or otherwise minimize patient trauma. Depending on the particular application, energy-based surgical instruments may utilize different surgical technologies including, for example, ultrasonic and/or electro-surgical (e.g., radio frequency (RF)) technologies.

A typical ultrasonic surgical instrument may include a handpiece containing an ultrasonic transducer and an elongated shaft assembly having a distally mounted end effector to effect the cutting and sealing of tissue. For example, the end effector may include a jaw assembly having an ultrasonic blade and a clamp arm, which may include a non-stick tissue pad or similar bed to receive the ultrasonic blade. In some cases, the elongated shaft assembly may be permanently affixed to the handpiece. In other cases, the elongated shaft assembly may be detachable from the handpiece, as in the case of a disposable shaft assembly or a shaft assembly that is interchangeable between different handpieces. In use, the end effector transmits ultrasonic energy to tissue brought into contact with the ultrasonic blade of the end effector to realize the cutting and sealing action. Such ultrasonic surgical devices may be configured for open surgical use, laparoscopic, and/or endoscopic surgical procedures including robotic-assisted procedures.

Ultrasonic energy cuts and coagulates tissue using temperatures lower than those used in electro-surgical procedures. Vibrating at high frequencies (e.g., 55,500 times per second), the ultrasonic blade denatures protein in the tissue to form a sticky coagulum. Pressure exerted on tissue by the ultrasonic blade surface collapses blood vessels and allows the coagulum to form a hemostatic seal. A surgeon can control the cutting speed and coagulation by the force applied to the tissue by the end effector, the time over which the force is applied, and the selected excursion level of the end effector.

In electro-surgical instruments, one or more electrodes are incorporated into the end effector and configured to apply therapeutic electrical current to the patient's tissue to create a hemostatic seal. In electro-surgical instruments that do not include a harmonic mode (i.e., do not include a harmonic blade), the end effector may be embodied as two clamp arms or jaws. In such embodiments, the electro-surgical instrument may include a separate mechanical knife or blade for cutting the tissue after the creation of the hemostatic seal, which may be incorporated into the elongated shaft attached to the end effector. In bi-polar embodiments, an active electrode may be attached to one of the clamp arms of the end effector and configured to introduce an electrical current into the tissue, which is received by a return electrode attached to the other clamp arm of the end effector (or as the blade itself in embodiments including a harmonic mode). Conversely, in mono-polar embodiments, the return electrode (e.g., a "grounding pad") may be separate from the electro-surgical instrument and located on a different part of the body of the patient. In some embodiments, the electro-surgical instrument may also be configured to apply a sub-therapeutic electrical current to the patient's tissue, which may be used for sensing purposes (e.g., measuring tissue impedance).

Electro-surgery forms hemostatic seals by generating heat in the tissue via the introduced electrical energy, which is embodied as radio frequency ("RF") energy. The particular frequency employed can vary based on the intended use of the electro-surgical instrument within the range of about 100 kHz to 1 MHz, although higher frequencies can be employed in some embodiments. Additionally, sub-therapeutic frequencies may be used in some situations for purposes other than hemostatic sealing, such as performing various electrical measurements on the tissue.

It should be appreciated that some energy-based surgical instruments may employ dual or multi-modal technologies for the transection and/or hemostasis of patient tissue. For example, in some cases, an energy-based surgical instrument may include both ultrasonic and electro-surgical capabilities (e.g., by utilizing the ultrasonic blade as an electrode for the electro-surgery mode), which increases the surgical options provided by the surgical instrument to the surgeon.

### SUMMARY

According to one aspect of the disclosure, a method for controlling an energy-based surgical instrument includes determining, by a control element, an instrument state of the energy-based surgical instrument; determining, by the control element, a feedback indication based on the instrument state, wherein the feedback indication is indicative of the instrument state and is interpretable by a user of the surgical instrument; and outputting, by the control element, the feedback indication with a feedback device.

In some embodiments, determining the instrument state includes determining an activation mode for the surgical instrument, wherein the activation mode comprises automatic mode or latch mode. In some embodiments, determining the instrument state includes determining an energy modality for the surgical instrument, wherein the energy modality comprises ultrasound or radio frequency (RF). In some embodiments, determining the instrument state includes determining a requested activation status based on a user input. In some embodiments, the user input has a range of actuation states from not actuated to fully actuated. In some embodiments, determining the instrument state includes determining an operational status of the surgical instrument based on instrument data. In some embodiments, the operational status comprises energy deactivated or energy activated. In some embodiments, the instrument data comprises sensor data indicative of tension applied to an end effector of the surgical instrument.

In some embodiments, outputting the feedback indication includes activating a visual indicator coupled to a handpiece of the surgical instrument. In some embodiments, outputting the feedback indication includes displaying the feedback indication with a display device, wherein the display device comprises a display of a surgical generator coupled to the surgical instrument or a laproscopic monitor in communication with the surgical instrument. In some embodiments, outputting the feedback indication includes outputting an audible indicator with an audio transducer of a surgical generator coupled to the surgical instrument. In some embodiments, outputting the feedback indication includes generating haptic feedback with a haptic device coupled to a handpiece of the surgical instrument.

In some embodiments, determining the instrument state includes determining an activation mode for the surgical instrument based on a mode selector switch of the surgical instrument, wherein the activation mode comprises automatic mode or latch mode; determining the feedback indication includes determining a first tone when the activation mode comprises the automatic mode and a second tone when the activation mode comprises the latch mode; and outputting the feedback indication includes outputting the first tone with an audio transducer of a surgical generator coupled to the surgical instrument when the activation mode comprises the automatic mode and outputting the second tone with the audio transducer of the surgical generator when the activation mode comprises the latch mode. In some embodiments, outputting the feedback indication further includes activating a visual indicator coupled to the surgical instrument, wherein the visual indicator is indicative of the activation mode.

In some embodiments, determining the instrument state comprises determining an activation mode for the surgical instrument based on a mode selector switch of the surgical instrument, wherein the activation mode comprises automatic mode or latch mode; determining the feedback indication includes determining a first haptic pattern when the activation mode comprises the automatic mode and a second haptic pattern when the activation mode comprises the latch mode; and outputting the feedback indication includes outputting the first haptic pattern or the second haptic pattern with a haptic device coupled to the surgical instrument.

In some embodiments, determining the instrument state includes determining a requested activation status based on an actuation state of a user input, wherein the user input is operatable over a range of actuation from not actuated to fully actuated; determining the feedback indication includes determining a first haptic pattern when the actuation state of the user input reaches a first position between not actuated and fully actuated; and determining a second haptic pattern when the actuation state of the user input reaches fully actuated; and outputting the feedback indication includes outputting the first haptic pattern or the second haptic pattern with a haptic device coupled to the surgical instrument. In some embodiments, the user input comprises a primary trigger of the surgical instrument. In some embodiments, the method further includes activating, by the control element, energy delivery with the surgical instrument simultaneously with outputting the second haptic pattern when the actuation state of the user input reaches fully actuated. In some embodiments, outputting the feedback indication includes outputting the second haptic pattern sequentially after outputting the first haptic pattern.

In some embodiments, determining the instrument state includes measuring tension on an end effector of the surgical instrument while applying ultrasound energy with the end effector; determining the feedback indication includes determining a first haptic pattern based on the measured tension; and outputting the feedback indication includes outputting the first haptic pattern with a haptic device coupled to the surgical instrument. In some embodiments, determining the first haptic pattern based on the measured tension includes increasing a frequency of haptic feedback pulses as an amount of the measured tension increases. In some embodiments, determining the first haptic pattern based on the measured tension includes decreasing a frequency of haptic feedback pulses as an amount of the measured tension nears a predetermined amount of tension. In some embodiments, the predetermined amount of tension is conducive for hemostasis.

According to another aspect, a system for controlling an energy-based surgical instrument includes the energy-based surgical instrument, a feedback device coupled to the surgical instrument, and a control element. The energy-based surgical instrument includes an end effector. The control element is configured to determine an instrument state of the energy-based surgical instrument, determine a feedback indication based on the instrument state, wherein the feedback indication is indicative of the instrument state and is interpretable by a user of the surgical instrument, and output the feedback indication with the feedback device.

In some embodiments, the instrument state comprises an activation mode for the surgical instrument, wherein the activation mode comprises automatic mode or latch mode. In some embodiments, the instrument state comprises an energy modality for the surgical instrument, wherein the energy modality comprises ultrasound or radio frequency (RF). In some embodiments, the instrument state comprises a requested activation status based on a user input. In some embodiments, the user input has a range of actuation states from not actuated to fully actuated. In some embodiments, the instrument state comprises an operational status of the surgical instrument based on instrument data. In some embodiments, the operational status comprises energy deactivated or energy activated. In some embodiments, the instrument data comprises sensor data indicative of tension applied to an end effector of the surgical instrument.

In some embodiments, to output the feedback indication includes to activate a visual indicator coupled to a handpiece of the surgical instrument. In some embodiments, to output the feedback indication includes to display the feedback indication with a display device, wherein the display device comprises a display of a surgical generator coupled to the surgical instrument or a laproscopic monitor in communication with the surgical instrument. In some embodiments, to output the feedback indication includes to output an audible indicator with an audio transducer of a surgical generator coupled to the surgical instrument. In some embodiments, the energy-based surgical instrument includes a haptic device coupled to a handpiece of the surgical instrument, and to output the feedback indication includes to generate haptic feedback with the haptic device.

In some embodiments, to determine the instrument state includes to determine an activation mode for the surgical instrument based on a mode selector switch of the surgical instrument, wherein the activation mode comprises automatic mode or latch mode; to determine the feedback indication includes to determine a first tone when the activation mode comprises the automatic mode and a second tone when the activation mode comprises the latch mode; and to output the feedback indication includes to output the first tone with an audio transducer of a surgical generator coupled to the surgical instrument when the activation mode comprises the automatic mode and to output the second tone with the audio transducer of the surgical generator when the activation mode comprises the latch mode. In some embodiments, to output the feedback indication further includes to activate a visual indicator coupled to the surgical instrument, wherein the visual indicator is indicative of the activation mode.

In some embodiments, to determine the instrument state includes to determine an activation mode for the surgical instrument based on a mode selector switch of the surgical instrument, wherein the activation mode comprises automatic mode or latch mode; to determine the feedback indication includes to determine a first haptic pattern when the activation mode comprises the automatic mode and a second haptic pattern when the activation mode comprises the latch mode; and to output the feedback indication includes to output the first haptic pattern or the second haptic pattern with a haptic device coupled to the surgical instrument.

In some embodiments, to determine the instrument state includes to determine a requested activation status based on an actuation state of a user input, wherein the user input is operatable over a range of actuation from not actuated to fully actuated; to determine the feedback indication includes to determine a first haptic pattern when the actuation state of the user input reaches a first position between not actuated and fully actuated; and to determine a second haptic pattern when the actuation state of the user input reaches fully actuated; and to output the feedback indication includes to output the first haptic pattern or the second haptic pattern with a haptic device coupled to the surgical instrument. In some embodiments, the user input comprises a primary trigger of the surgical instrument. In some embodiments, the control element is further configured to activate energy delivery with the surgical instrument simultaneously with output of the second haptic pattern when the actuation state of the user input reaches fully actuated. In some embodiments, to output the feedback indication includes to output the second haptic pattern sequentially after output of the first haptic pattern.

In some embodiments, to determine the instrument state includes to measure tension on an end effector of the surgical instrument during application of ultrasound energy with the end effector; to determine the feedback indication includes to determine a first haptic pattern based on the measured tension; and to output the feedback indication includes to output the first haptic pattern with a haptic device coupled to the surgical instrument. In some embodiments, to determine the first haptic pattern based on the measured tension includes to increase a frequency of haptic feedback pulses as an amount of the measured tension increases. In some embodiments, to determine the first haptic pattern based on the measured tension includes to decrease a frequency of haptic feedback pulses as an amount of the measured tension nears a predetermined amount of tension. In some embodiments, the predetermined amount of tension is conducive for hemostasis.

### BRIEF DESCRIPTION OF THE DRAWINGS

The detailed description particularly refers to the following figures, in which:
FIG. 1 is a simplified diagram of an embodiment of a system for performing an energy-based surgical procedure;
FIG. 2 is a perspective view of an embodiment of an energy-based surgical instrument of the system of FIG. 1;
FIG. 3 is a side elevation view of a jaw assembly of an end effector of the surgical instrument of FIG. 2 including an ultrasonic blade and in an open state;
FIG. 4 is a side elevation view of the jaw assembly of the end effector of the surgical instrument of FIG. 2 including an ultrasonic blade and in a closed state;
FIG. 5A is a perspective view of another embodiment of the end effector of the surgical instrument of FIG. 2 including an electrode on a lower jaw clamp of the jaw assembly;
FIG. 5B is a perspective view of another embodiment of the end effector of the surgical instrument of FIG. 2 including two jaw clamps, each having an electrode attached thereto;
FIG. 6 is an exploded view of the surgical instrument of FIG. 2;
FIG. 7 is a block diagram of a control circuit of the surgical instrument of FIG. 2;
FIG. 8 is a schematic diagram of an energy-based surgical instrument system with multiple feedback modalities;
FIG. 9 is a simplified flow diagram of a method for providing feedback on instrument state for a multi-mode energy-based surgical instrument;
FIG. 10 is a simplified flow diagram of a method for providing user feedback for activation of cutting energy for an energy-based surgical instrument; and
FIG. 11 is a simplified flow diagram of a method for providing haptic feedback during activation of an energy-based surgical instrument.

### DETAILED DESCRIPTION OF THE DRAWINGS

While the concepts of the present disclosure are susceptible to various modifications and alternative forms, specific illustrative embodiments thereof have been shown by way of example in the drawings and will herein be described in detail. It should be understood, however, that there is no intent to limit the concepts of the present disclosure to the particular forms disclosed, but on the contrary, the intention is to cover all modifications, equivalents, and alternatives falling within the spirit and scope of the invention as defined by the appended claims.

Terms representing anatomical references, such as anterior, posterior, medial, lateral, superior, inferior, distal, proximal, et cetera, may be used throughout the specification in reference to the surgical instruments described herein as well as in reference to the patient's natural anatomy. Such terms have well-understood meanings in both the study of anatomy and the field of surgery. Use of such anatomical reference terms in the written description and claims is intended to be consistent with their well-understood meanings unless noted otherwise.

References in the specification to "one embodiment," "an embodiment," "an illustrative embodiment," etc., indicate that the embodiment described may include a particular feature, structure, or characteristic, but every embodiment may or may not necessarily include that particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to effect such feature, structure, or characteristic in connection with other embodiments whether or not explicitly described. Additionally, it should be appreciated that items included in a list in the form of "at least one A, B, and C" can mean (A); (B); (C); (A and B); (A and C); (B and C); or (A, B, and C). Similarly, items listed in the form of "at least one of A, B, or C" can mean (A); (B); (C); (A and B); (A and C); (B and C); or (A, B, and C).

The disclosed embodiments may be implemented, in some cases, in hardware, firmware, software, or any combination thereof. The disclosed embodiments may also be implemented as instructions carried by or stored on a transitory or non-transitory machine-readable (e.g., computer-readable) storage medium, which may be read and executed by one or more processors. A machine-readable storage medium may be embodied as any storage device, mechanism, or other physical structure for storing or transmitting information in a form readable by a machine (e.g., a volatile or non-volatile memory, a media disc, or other media device).

In the drawings, some structural or method features may be shown in specific arrangements and/or orderings. However, it should be appreciated that such specific arrangements and/or orderings may not be required. Rather, in some embodiments, such features may be arranged in a different manner and/or order than shown in the illustrative figures. Additionally, the inclusion of a structural or method feature in a particular figure is not meant to imply that such feature is required in all embodiments and, in some embodiments, may not be included or may be combined with other features.

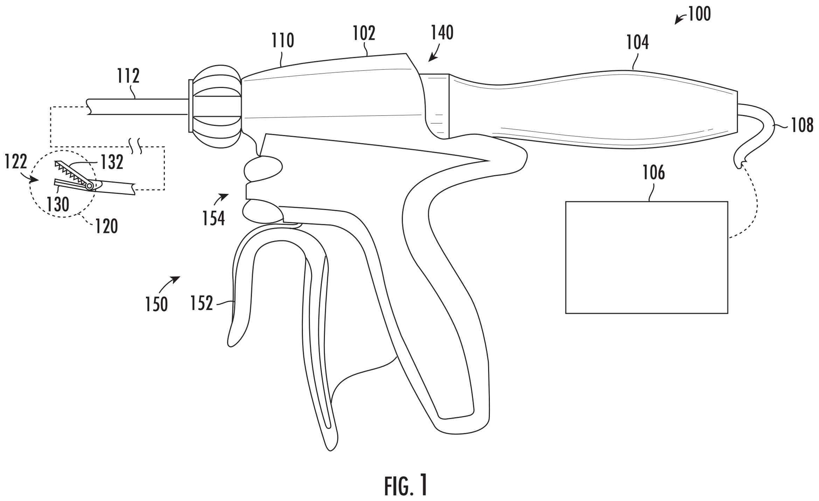

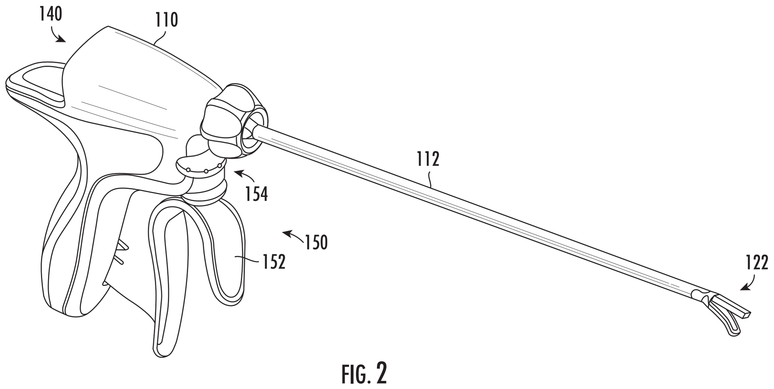

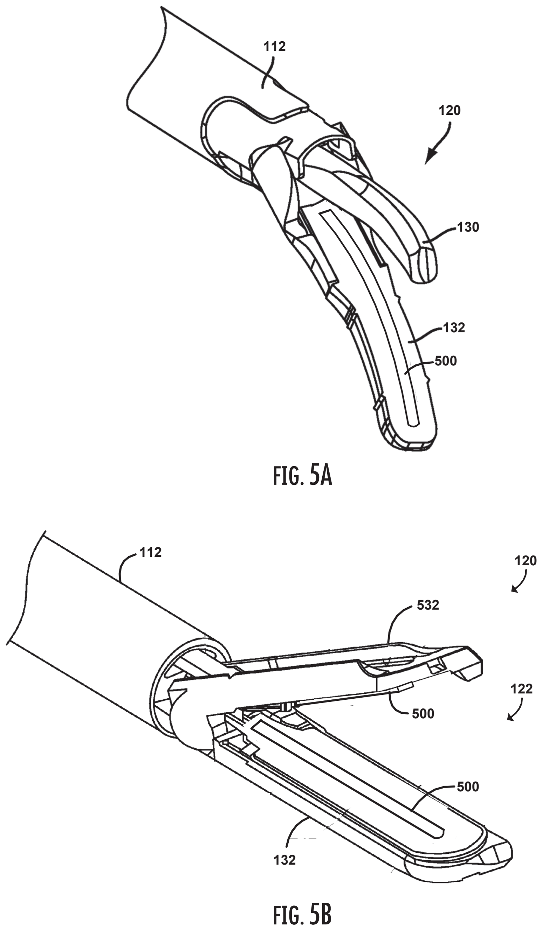

Referring now to FIGS. 1 and 2, in an illustrative embodiment, a system 100 for performing an energy-based surgical procedure includes a surgical instrument 102, a transducer 104, and a generator 106. The surgical instrument 102 is illustratively embodied as an ultrasonic surgical instrument, but may be embodied as an electro-surgical surgical instrument or a multi-modal, ultrasonic/elector-surgical surgical instrument in other embodiments. In use, the surgical instrument 102 is usable to perform various surgical procedures including laparoscopic, endoscopic, or traditional open surgical procedures. In doing so, a surgeon may selectively activate an ultrasonic mode (and/or an electro-surgical/RF mode) of the surgical instrument 102. In the ultrasonic mode, the generator 106 drives the transducer 104 to cause an ultrasonic blade 130 of a jaw assembly 122 of an end effector 120 of the surgical instrument 102 to vibrate at a reference frequency, which facilitates the contemporaneous cutting and hemostatic sealing of patient tissue. Additionally or alternatively, in some embodiments, the surgeon may selectively activate an electro-surgical mode of the surgical instrument 102 to deliver an amount of therapeutic RF energy to the patient tissue to effect hemostatic sealing. In such embodiments, the blade 130 may be embodied as an ultrasonic blade 130 or as a mechanical blade designed to cut tissue using mechanical force (e.g., in those embodiments not employing ultrasonic technologies). Furthermore, in some embodiments, the surgical instrument 102 may be configured with only an electro-surgical/RF mode and, in such embodiments, the jaw assembly 122 of the end effector 120 may not include the ultrasonic blade 130 as discussed in more detail below in regard to FIG. 5B.

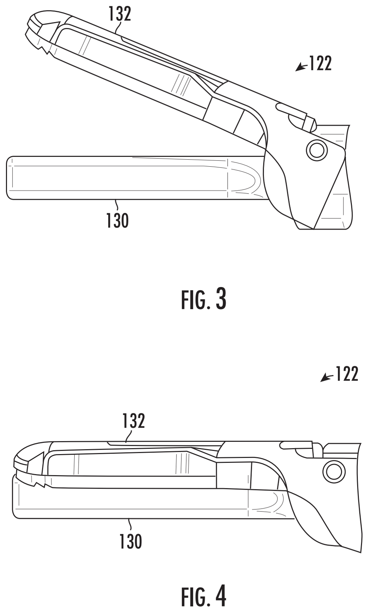

The surgical instrument 102 is illustratively embodied as ultrasonic surgical shears but may be embodied as other types of surgical instruments having an ultrasonic mode and/or electro-surgical mode in other embodiments. In the illustrative embodiment, the surgical instrument 102 includes a handle assembly 110 and an elongated shaft assembly 112, which extends distally away from the handle assembly 110 and may be removably attached to the handle assembly 110 in some embodiments. The elongated shaft assembly 112 includes the end effector 120 located at a distal end opposite the handle assembly 110. The end effector 120 includes the jaw assembly 122, which illustratively includes the ultrasonic blade 130 and a corresponding jaw clamp 132 (but may include two jaw clamps in those embodiments having only an electro-surgical/RF mode). As shown in FIGS. 3 and 4, the jaw assembly 122 is movable between an open state (FIG. 3) in which the jaw clamp 132 is positioned away from the ultrasonic blade 130 and a closed state (FIG. 4) in which the jaw clamp 132 is positioned near or otherwise contacts the ultrasonic blade 130. Actuation of the jaw assembly 122 from the open state to the closed state allows for the grasping, cutting, and coagulation of vessels and/or tissue by the jaw assembly 122. It should be appreciated that the open state may correspond to a degree of openness that is less than a fully opened position of the jaw assembly 122 and the closed state may correspond to a degree of closeness that is less than a fully closed position. That is, the closed state may, for example correspond to a minimal distance between the distal ends of the jaw clamp 132 and the ultrasonic blade 130 and the open state may correspond to a maximum distance between the distal ends of the jaw clamp 132 and the ultrasonic blade 130. However, in other embodiments, the open state may correspond to a fully opened position of the jaw assembly 122 and the closed state may correspond to a fully closed position of the jaw assembly 122.

In those embodiments in which the surgical instrument 102 includes both a ultrasonic mode and an electro-surgical/RF mode, the end effector 120 may include one or more RF electrodes 500 incorporated into the jaw clamp 132 as shown in FIG. 5A. Although the illustrative end effector 120 includes only a single electrode 500 in the embodiment of FIG. 5A, it should be appreciated that the end effector 120 may include additional electrodes 500 in other embodiments (e.g., multiple pads of electrodes 500). The electrode(s) 500 may be embodied as an active electrode configured to the RF energy or as a return electrode configured to "sink" an applied RF energy. In those embodiments utilizing bi-polar RF implementation, the ultrasonic blade 130 may embody the active or return electrode, with the electrode 500 embodying the other active or return electrode. Alternatively, other active or return electrodes may be incorporated on the ultrasonic blade 130 or in another part of the jaw assembly 122 of the end effector 120. In mono-polar implementation, the RF electrode(s) 500 may be embodied as an active electrode, and a return electrode may be attached to a portion of the patient's body.

In those embodiments in which the surgical instrument 102 includes only an electro-surgical/RF mode, the jaw assembly 122 of the end effector 120 includes a jaw clamp 532 in place of the ultrasonic blade 130 as shown in FIG. 5B. In such embodiments, an electrode 500 may be attached to or otherwise incorporated into each jaw clamp 132, 532 and be embodied as an active or a return electrode to facilitate the application of RF energy to tissue captured between the jaw clamps 132, 532. In such embodiments, the surgical instrument 102 may include a knife incorporated into the elongated shaft assembly 112 that is configured to eject outwardly to cut the patient's tissue after sealing of the tissue by the RF energy.

Referring back to FIGS. 1 and 2, in those embodiments including ultrasonic capabilities, the handle assembly 110 includes a receptacle 140 configured to receive the transducer 104 to facilitate connection of the transducer 104 to the handle assembly 110 and the elongated shaft assembly 112. The handle assembly 110 also includes a trigger assembly 150, which includes a primary trigger 152 and a switch assembly 154. The primary trigger 152 is operable by the surgeon to move the jaw assembly 122 of the end effector 120 between the open and closed states. The switch assembly 154 includes one or more buttons, which are selectable by the surgeon to activate (and configure, in some embodiments) the ultrasonic mode and/or the electro-surgical mode of the surgical instrument 102.

The transducer 104 is illustratively connected to the generator 106 by a cable assembly 108. As discussed above, the generator 106 is configured to drive the transducer 104 at a reference or resonant frequency to thereby cause the ultrasonic blade 130 to vibrate. For example, in an illustrative embodiment, the generator 106 may supply an electrical signal to the transducer 104 to cause the ultrasonic blade 130 of the jaw assembly 122 to vibrate longitudinally in the range of, for example, approximately 20 kHz to 250 kHz. In particular embodiments, for example, the ultrasonic blade 130 may vibrate in the range of about 54 kHz to 56 kHz (e.g., at about 55.5 kHz). In other embodiments, the ultrasonic blade 130 may vibrate at other frequencies including, for example, about 31 kHz or about 80 kHz. The excursion of the vibrations at the ultrasonic blade 130 can be controlled by, for example, controlling the amplitude of the electrical signal applied to the transducer 104 by the generator 106. The generator 106 may be activated so that electrical energy may be continuously or intermittently supplied to the transducer 104. The generator 106 also has a power line (not shown) for insertion in an electro-surgical unit or conventional electrical outlet. Additionally or alternatively, the generator 106 may be powered by a direct current (DC) source, such as a battery.

In some embodiments, the generator 106 may be configured to operate in different modes. In such embodiments, the generator 106 may include an ultrasonic generator module 162 for controlling an ultrasonic mode, an electro-surgical/Radio Frequency (RF) generator module 164 for controlling an electro-surgical mode, and/or other generator modules (e.g., a heat generator module) for controlling other operation modes. The various modes of the generator 106 may be operated independently of each other in some embodiments. For example, the generator 106 may activate the ultrasonic mode of the ultrasonic generator module 162 to apply ultrasonic energy to the jaw assembly 122 and subsequently, either therapeutic or sub-therapeutic RF energy may be applied to the jaw assembly 122 by the electro-surgical generator module 164. Alternatively, the activation modes of the generator 106 may be operated simultaneously or contemporaneously with each other.

In the electro-surgical mode, the electro-surgical generator module 164 is configured to generate RF energy at a frequency in the range of about 100 kilohertz (100 kHz) to about 1 megahertz (1 MHz). The generated RF energy is supplied to the patient's tissue via the electrodes 500 of the end effector 120 as described above in regard to FIG. 5. In some embodiments, the electro-surgical generator module 164 may also be configured to selectively provide the RF energy at sub-therapeutic levels to perform various electrical measurements of the patient's tissue. For example, the electro-surgical generator module 164 may be configured to measure an impedance of the patient's tissue using the electrodes 500 and a suitable RF energy level.

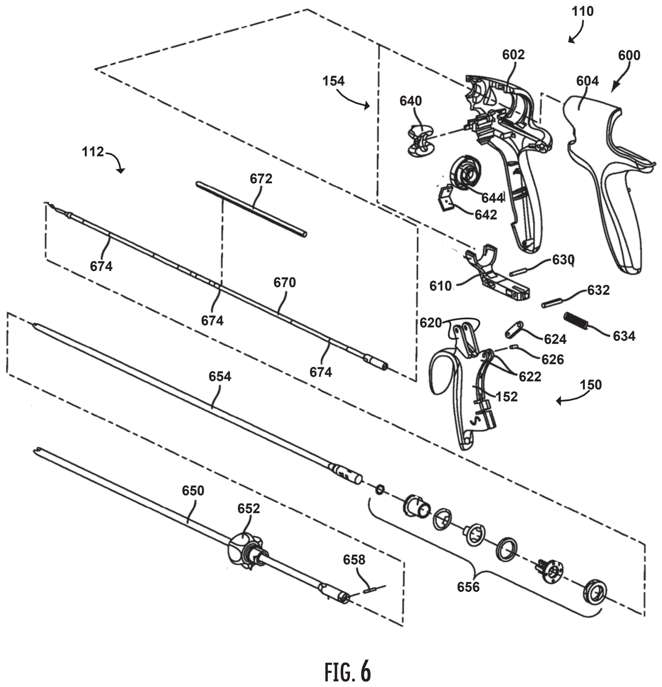

Referring now to FIG. 6, as discussed above, the illustrative surgical instrument 102 includes the handle assembly 110 and the elongated shaft assembly 112, which extends distally away from the handle assembly 110. The handle assembly 110 includes a housing 600, which includes a right half-housing 602 and a left half-housing 604. The half-housings 602, 604 are configured to mate with each other to form the housing 600. To facilitate such mating, each of the half-housings 602, 604 may include various interfaces sized to mechanically align and engage one another to form the housing 600 and enclose the internal working components of the surgical instrument 102.

The primary trigger 152 of the trigger assembly 150 is coupled to a linkage mechanism to translate the rotational motion of the primary trigger 152 to axial motion of a yoke 610, which in turn is configured to move the jaw assembly 122 of the end effector 120 between the open and closed states via the elongated shaft assembly 112. The primary trigger 152 includes a first set of flanges 620 having openings formed therein to receive a first yoke pin 630, which extends through the yoke 610. The primary trigger 152 also includes a second set of flanges 622 configured to receive a first end of a link 624. A trigger pin 626 is received in openings formed in the first end of the link 624 and the second set of flanges 622. The trigger pin 626 forms a trigger pivot point for the primary trigger 152. A second end of the link 624, opposite the first end, is received in a slot formed in a proximal end of the yoke 610 and retained therein by a second yoke pin 632. As the primary trigger 152 is rotated about the pivot point formed from the trigger pin 626, the yoke 610 translates horizontally. A spring 634 is used to bias the yoke forward such that the jaw assembly 122 of the end effector 120 is biased to the open state (or a fully opened state).

As discussed above, the trigger assembly 150 also includes a switch assembly 154. The switch assembly 154 illustratively includes a toggle switch 640, which is selectable to activate one or more switches 642. Activation of the switches 642 electrically energizes an electrical element 644, which electrically energizes the ultrasonic transducer 104 to engage the ultrasonic mode of the surgical instrument 102.

The elongated shaft assembly 112 includes an outer tubular sheath 650 and a rotation knob 652 coupled to the outer cylindrical sheath 650. The rotation knob 652 is operable to rotate the outer cylindrical sheath 650 about an axis defined by the outer cylindrical sheath 650. A reciprocating tubular actuator 654 is located within the outer tubular sheath 650 and mechanically engaged with the end effector 120 on a distal end. The reciprocating tubular actuator 654 is also mechanically engaged, on a proximal end, with the yoke 610 within the handle assembly 110 via coupling elements 656. In embodiments including an ultrasonic mode, an ultrasonic waveguide 670 is located within the reciprocating tubular actuator 654. A distal end of the ultrasonic waveguide 670 is acoustically coupled (e.g., directly or indirectly mechanically coupled) to the ultrasonic blade 130, and a proximal end is acoustically coupled to the transducer 104. The ultrasonic waveguide 670 may be isolated from other components of the elongated shaft assembly 112 by a protective sheath 672 and a number of isolation elements 674. The outer tubular sheath 650, the reciprocating tubular actuator 654, and the ultrasonic waveguide 670 are mechanically engaged together via a pin 658.

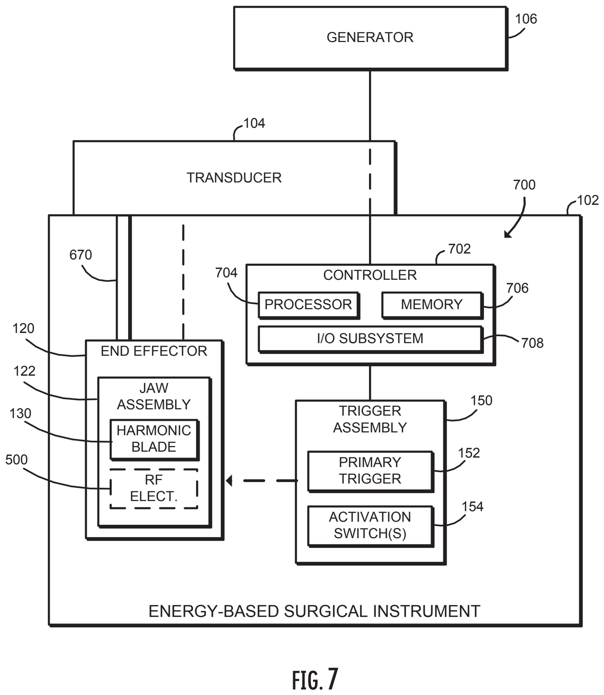

Referring now to FIG. 7, in the illustrative embodiment, the surgical instrument 102 includes a control circuit 700. The control circuit 700 includes a controller 702 and the trigger assembly 150, which cooperate to provide ultrasonic energy to the harmonic blade 130 of the jaw assembly 122 of the end effector 120 and/or RF energy to the RF electrodes 500 of the jaw assembly 122, depending on the operation modes of the surgical instrument 102 as discussed above. In other embodiments, however, the control circuit 700 may include additional or other electronic devices and/or circuit.

The controller 702 may be embodied as any type of controller, functional block, digital logic, or other component, device, circuitry, or collection thereof capable of performing the functions described herein. In illustrative embodiment, the controller 702 includes a processor 704, a memory 706, and an input/output (I/O) subsystem 708. The processor 704 may be embodied as any type of processor capable of performing the functions described herein. For example, the processor 704 may be embodied as a single or multi-core processor(s), digital signal processor, microcontroller, or other processor or processing/controlling circuit. Similarly, the memory 706 may be embodied as any type of volatile and/or non-volatile memory or data storage capable of performing the functions described herein. In operation, the memory 706 may store various data and software used during operation of the control circuit 700 such as executable firmware or software, programs, libraries, and drivers, which may be executed or otherwise used by the processor 704.

The processor 704 and memory 706 are communicatively coupled to other components of the control circuit 700 via the I/O subsystem 708, which may be embodied as circuitry and/or components to facilitate input/output operations between the controller 702 (e.g., the processor 704 and the memory 706) and the other components of the control circuit 700. For example, the I/O subsystem 708 may be embodied as, or otherwise include, memory controller hubs, input/output control hubs, firmware devices, communication links (i.e., point-to-point links, bus links, wires, cables, light guides, printed circuit board traces, etc.) and/or other components and subsystems to facilitate the input/output operations. In some embodiments, the I/O subsystem 708 may form a portion of a system-on-a-chip (SoC) and be incorporated, along with the processor 704 and the memory 706, and other components of the surgical instrument 102, on a single integrated circuit chip. Additionally, in some embodiments, the memory 706, or portions of the memory 706, may be incorporated into the processor 704.

During operation, as discussed above, the controller 702 is configured to control activation of an ultrasonic mode and/or an electro-surgical/RF mode of the surgical instrument 102. To do so, the controller 702 may monitor for activation of the primary trigger 152 and/or one or more activation switches 154 of the trigger assembly 150. In response to activation of the appropriate trigger 152 or switch 154, the controller 702 controls the transducer 104 to generate the ultrasonic energy, which is propagated to the harmonic blade 130 via the ultrasonic waveguide 670. Additionally or alternatively, in response to activation of a corresponding switch 154 of the trigger assembly 150, the controller 702 may be configured to supply an amount of RF energy, via the electro-surgical generator module 164 to the RF electrodes 500 via interconnections 710. It should be appreciated that, although the transducer 104 and the generator 106 are shown as separate components from the energy-based surgical instrument 102 in FIGS. 1 and 7, the transducer 104 and/or the generator 106 may be incorporated into the surgical instrument 102 in other embodiments.

Current harmonic and RF generators may generate a tone when activated and a separate tone when completed. However, in an operating room (OR) there are lots of beeping and tones being played by surgical instruments, and the repeating tone of the generator that changes may be hard to follow and is further keyed only to the activation of the energy mode and its competition. As devices become more sophisticated, there are more aspects to the device than the progression of the energy applied. Additionally, as motorized clamping mechanisms and automatic functions are integrated into surgical instruments 102, there may be other feedback provided, such as sufficient clamp force, inabilities of the system to actuate for a variety of reasons, or even full cycle or full knife stokes, which were entirely mechanically coupled in the past. The illustrative system 100 may provide such local feedbacks, which may be communicated on the handle or local to the handle to provide the feedback. Robotics may also need local feedback. As described further below, feedback may be electrical or generated through mechanical mechanisms like a zip tie or ratcheting device.

In some embodiments, the system 100 may provide user feedback on the energy mode and transition state for completion or selection of cutting. For example, a user may be provided feedback on and aspect of the activation stat or energy mobility that is or will be provided to the end-effector. The feedback could be provided during or before the activation of energy indicating the type of energy, the activation sequence, or the effect the energy will provide (e.g., sealing or cutting or a combination thereof). In an embodiment, the feedback may be an audible or tactile indicator of impending energy activation. Impending actions or energy mode changes may be due to "automated" actions that happen in series or as a result of a previous user actuation. In an embodiment, the visual or audible feedback may be used to inform the user if the device will cut or not cut tissue while also coagulating tissue. In an embodiment, the feedback could inform the user which type of energy (e.g., ultrasound or RF) is going to be provided.

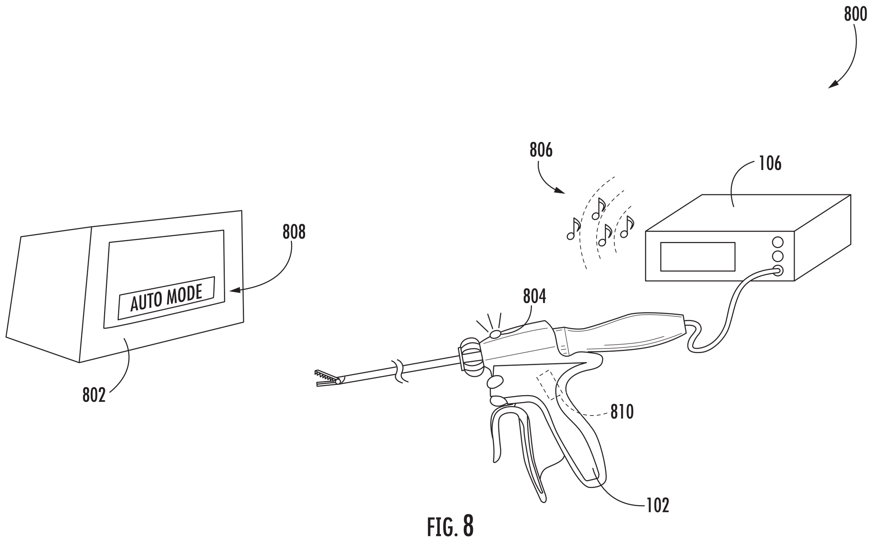

Referring now to FIG. 8, in another illustrative embodiment, a system 800 includes a surgical instrument 102, a generator 106, and in some embodiments, a laparoscopic monitor 802. The surgical instrument 102 supports two modes of operation for application of surgical energy. In a latch mode, the surgeon or other user pulls the primary trigger 152, which moves the jaw assembly 122 of the end effector 120 into the closed state. When the jaw assembly 122 is in the closed state, a latch mechanism locks the primary trigger 152 in place, holding the jaw assembly 122 in the closed state. When the primary trigger 152 is latched in place, the surgeon may operate one or more buttons of the switch assembly 154 in order to activate delivery of energy (e.g., ultrasound or RF energy). If the button of the switch assembly 154 is activated when the primary trigger 152 is not latched (e.g., when the jaw assembly 122 is in the open or a partially closed state), the energy may not be activated (i.e., the surgical instrument 102 may be locked out).

In an automatic or non-latch mode, the primary trigger 152 may not have a latch mechanism, or the latch mechanism may be disabled. When the surgeon pulls the primary trigger 152 to a fully activated position, delivery of energy is activated (e.g., ultrasound or RF energy). In the automatic or non-latch mode, the surgeon may not be required to independently activate buttons of the switch assembly 154 in order to activate energy.

In the illustrative embodiment of FIG. 8, which the latch mode or automatic (non-latch) mode is set, a sensor (such as a proximity sensor or optical sensor) or an electrical contact of the instrument 102 sends a message signal to the generator 106. The generator 106 may provide an audible tone 806 to denote when the instrument 102 is in automatic (non-latch) mode, and a different audible tone 806 when taken out of automatic mode (e.g., into latch mode).

Additionally or alternatively, in combination with or without the audible tone 806, in some embodiments the surgical instrument 102 may include a visible indicator 804 such as an LED light, which indicates whether the automatic mode is active (e.g., LED on) or if the instrument 102 is in the latch mode (e.g., LED off).

Additionally or alternatively, in combination with or without the audible signal 806 and/or the visible indicator 804, in some embodiments, a message may be sent from the generator 106 for display on a generator screen and/or on the laparoscopic monitor 802. Either the monitor 802, the generator screen, and/or other display device may display a visible indication 808 of the selected device 102 mode (e.g., latch mode or automatic (non-latch) mode). Accordingly, the system 800 may provide improved feedback to a user indicative of the mode of the device 102, allowing for intuitive and streamlined indications of the operating mode of the instrument 102.

Additionally or alternatively, in combination with or without the audible signal 806, the visible indicator 804, and/or the display screen of the generator 106 or the laparoscopic monitor 802, in some embodiments a message may be sent from the generator 106 to activate a haptic device 810 included in a handpiece of the surgical instrument 102. The haptic device 810 may be embodied as any device configured to generate vibrations, tactile sensations, or other feedback that may be sensed by a user of the surgical instrument 102 using senses of touch and/or proprioception. For example, the haptic device 810 may be embodied as an electric motor with eccentric rotating mass. In other embodiments, the haptic device 810 may be a linear resonant actuator, a piezoelectric actuator, a linear magnetic ram motor, or other device capable of generating haptic feedback. Additionally or alternatively, rather than being activated by a signal from the generator 106, in some embodiments the haptic device 810 may be activated by one or more signals from the controller 702 or other control element of the surgical instrument 102.

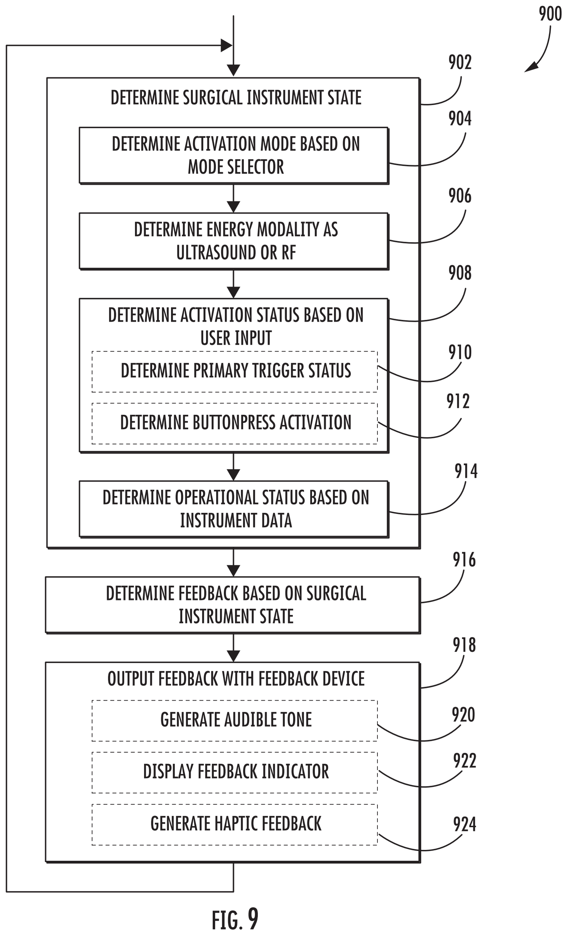

Referring now to FIG. 9, a method 900 for providing feedback on instrument state for a surgical instrument 102 is shown. The method 900 may be executed by the controller 702, the generator 106, and/or one or more other microcontrollers or other control elements of the system 100. The method 900 begins in block 902, in which the control element determines a current surgical instrument state for the surgical instrument 102. The surgical instrument state may include, for example, a currently activated mode of operation, energy modality, or other attributes of the surgical instrument 102, a currently requested activation status based on one or more user inputs provided by a surgeon or other user, a current operational status of the surgical instrument 102, or other attributes of the surgical instrument 102 and/or the environment of the surgical instrument 102.

In block 904, the control element determines an activation mode based on a mode selector of the surgical instrument 102. In the illustrative embodiment, the surgical instrument supports two activation modes: automatic mode and latch mode (also called manual mode). As described above, in the latch or manual mode, the surgeon or other user pulls the primary trigger 152, which moves the jaw assembly 122 of the end effector 120 into the closed state. When the jaw assembly 122 is in the closed state, a latch mechanism locks the primary trigger 152 in place, holding the jaw assembly 122 in the closed state. When the primary trigger 152 is latched in place, the surgeon may operate one or more buttons of the switch assembly 154 in order to activate delivery of energy (e.g., ultrasound or RF energy). If the button of the switch assembly 154 is activated when the primary trigger 152 is not latched (e.g., when the jaw assembly 122 is in the open or a partially closed state), the energy may not be activated (i.e., the surgical instrument 102 may be locked out).

In the automatic or non-latch mode, the primary trigger 152 may not have a latch mechanism, or the latch mechanism may be disabled. When the surgeon pulls the primary trigger 152 to a fully activated position, delivery of energy is activated (e.g., ultrasound or RF energy). In the automatic or non-latch mode, the surgeon may not be required to independently activate buttons of the switch assembly 154 in order to activate energy.

The surgical instrument 102 may include a mode selector, such as a mode selector switch or other input included in the switch assembly 154. The control element uses a sensor (e.g., a proximity sensor or optical sensor), an electric contact, or other technique to determine the position of the mode selector.

In block 906, the control element determines the current energy modality of the surgical instrument 102. Illustratively, the surgical instrument 102 may deliver ultrasound energy or radio frequency (RF) energy. The control element may determine which of those energy modalities will be delivered in response to fully activating the primary trigger 152. The current energy modality may be selected by the user, for example using an energy delivery selector switch and/or one or more buttons of the switch assembly 154.

In block 908, the control element determines a requested activation status based on user input to the surgical instrument. The requested activation status may include whether the user has actuated or partially actuated a user input that controls delivery of energy. In some embodiments, in block 910 the control element determines status of the primary trigger 152. The primary trigger 152 may be moveable from an initial position, in which the primary trigger 152 is not actuated, to a fully actuated position, in which the primary trigger 152 is pulled completely or otherwise pulled past a predetermined position, for example to a physical stop or other stopping position. The control element may determine the position of the primary trigger 152, including whether the primary trigger 152 is not actuated, fully actuated, or partially actuated, and in some embodiments may determine the relative position of the primary trigger 152 (for example determining a percentage of fully actuated or other indication of to which position the primary trigger 152 has been pulled). The control element may use any combination of sensors (e.g., proximity sensors, optical sensors, etc.) and/or electrical contacts to determine the location of the primary trigger 152. In some embodiments, in block 912 the control element may determine other buttonpress activations, for example with one or more buttons of the switch assembly 154.

In block 914, the control element determines operational status of the surgical instrument 102 based on instrument data related to the surgical instrument 102. For example, the control element may determine an energy delivery status; that is, whether the surgical instrument 102 is actively delivering energy, which energy modality is active, the current power level of energy delivery, or other energy delivery parameters. As another example, the control element may determine tension experienced by the end effector 120. For example, the control element may determine tension by measuring blade deflection of the end effector 120 using one or more sensors of the surgical instrument 102.

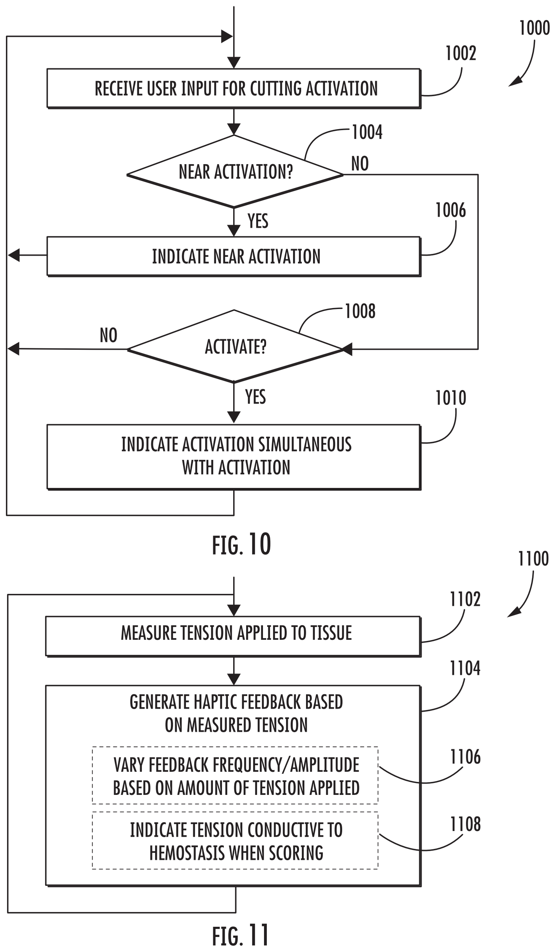

Although illustrated as determining each of the activation mode, the energy modality, the requested activation status, and the operational status, it should be understood that in embodiments the control element may determine any combination of those parameters of the surgical instrument state and/or other additional parameters of the surgical instrument state. Potential combinations of parameters that may be included in the surgical instrument state and associated feedback indications and feedback outputs are described further below in connection with the methods of FIGS. 10 and 11.

In block 916, the control element determines a feedback indication based on the determined surgical instrument state. The feedback indication may be any visual, audible, tactile, or other indication that is indicative of the surgical instrument state and is interpretable by the surgeon or other user of the surgical instrument 102. For example, as described above, the feedback indication may be indicative of the current activation mode of the surgical instrument 102 (e.g., automatic mode or latch mode). As another example, the feedback indication may be indicative of the current energy modality of the surgical instrument 102 (e.g., ultrasound and/or RF). As another example, the feedback indication may be indicative of the currently requested actuation status of the surgical instrument 102 (e.g., the current position of the primary trigger 152 or other user input). As another example, the feedback indication may be indicative of the current operational status of the surgical instrument 102, such as whether energy is actively being delivered, the tension currently applied to the ultrasonic blade 130, or other operational status. In some embodiments, the feedback indication may be a combination of one or more of those parameters of the surgical instrument state.

In block 918, the control element outputs the feedback indication using one or more feedback devices. In some embodiments, in block 920 the control element may generate an audible tone using an audio transducer, for example an audio transducer included in the surgical generator 106 or in the surgical instrument 102. In some embodiments, in block 922 the control element causes a visible indicator of the feedback indication to be displayed, for example with an LED light 804 or other visible indicator coupled to the surgical instrument 120. In some embodiments, the visible indicator of the feedback indication may be displayed on another device, such as a display screen of the generator 106 and/or a laparoscopic monitor 802.

In some embodiments, in block 922 the control element generates haptic feedback with the haptic device 810. The haptic feedback may include tactile bumps or other tactile signals. Additionally or alternatively, the haptic feedback may include haptic patterns or other pulses of tactile feedback. Attributes of the haptic feedback, including the amplitude and/or the frequency of haptic pulses, may be adjusted based on the feedback indication. For example, the frequency of haptic pulses may be proportional to the measured tension at the end effector 120 and/or a difference between the measured tension and a target tension value. Continuing that example, the surgeon may adjust tension on the end effector such that the frequency of the haptic feedback decreases, causing the tension on the end effector to reach a desired value (e.g., zero tension or other target tension value).

After outputting the feedback, the method 900 loops back to block 902 to continue determining surgical instrument state and outputting feedback based on that surgical instrument state. Accordingly, the system 100 provides improved feedback to a user indicative of the mode or other state of the surgical instrument 102, which allows for intuitive and streamlined indications of the operating mode of the instrument 102. Additionally, by providing haptic feedback to the surgeon concerning the operational mode of the surgical instrument 102 (e.g., whether automatic mode is activated and/or the current energy modality), the system 100 reduces cognitive load for the user and may improve safety as compared to systems that do not provide haptic feedback. For example, haptic feedback as described herein may notify the surgeon of the current operating mode of the surgical instrument 102 without requiring the surgeon to look away from the instrument to a separate display screen.

Referring now to FIG. 10, a method 1000 for controlling an energy-based surgical instrument 102 is shown. The method 1000 may be executed by the controller 702, the generator 106, and/or one or more other microcontrollers or other control elements of the system 100. The method 1000 begins in block 1002, in which the control element receives user input for cutting activation. The user input may include, for example, the position and/or activation state of the primary trigger 152 and/or one or more buttons or other controls of the switch assembly 154. As described above, in some embodiments, the user input received by the control element may indicate whether the primary trigger 152 is not actuated, fully actuated, or partially actuated, and may indicate at the current position of the primary trigger 152.

In block 1004, the control element determines whether the user input is near (i.e., just before) activation of cutting. For example, the control element may determine whether the user has moved an input device (e.g., primary trigger 152 or switch assembly 154) to a particular position, applied a particular amount of force, enabled or otherwise selected a cutting mode, or otherwise almost activated a cutting operation. In the illustrative embodiment, the control element determines whether the primary trigger 152 has been pulled to a predetermined position that is close to but not fully actuated. If cutting is not near activation (e.g., the user input is less than or greater than an amount or range of amounts that are near activation), the method 1000 branches ahead to block 1008, described below. If the user input is near activation of cutting, the method 1000 advances to block 1006.

In block 1006, the control element causes an indication that cutting is about to be activated. For example, the control element may generate haptic feedback, visual feedback, audible feedback, or otherwise indicate that cutting is about to be activated. For example, haptic feedback may activate just before activation of the ultrasonic blade 130 if the cutting mode is ultrasonic in addition to RF. This feedback may occur before pressure is adjusted for ultrasonic cutting. Accordingly, the system 100 provides feedback to the surgeon, indicating to the surgeon that if the primary trigger 152 is pulled further, ultrasonic energy will be applied. As described, the system 100 may provide haptic, tactile feedback without requiring a detent or other mechanism included in the primary trigger 152 mechanism. Further, this haptic feedback may not be performed if energy will not be applied when the trigger 152 is fully actuated (e.g., when the surgical instrument 102 is in latch or manual mode). Accordingly, the system 100 provides appropriate tactile feedback for multiple operating modes without requiring multiple mechanical tactile feedback systems and their associated costs. After indicating that cutting is about to start, the method 1000 loops back to block 1002 to continue monitoring user input.

In block 1008, the control element determines whether the user input indicates cutting should be activated. For example, the control element may determine whether the user has moved an input device (e.g., primary trigger 152 or switch assembly 154) past a particular position, applied more than a particular amount of force, or otherwise indicated that a cutting operation should be activated. If not (e.g., if the user input remains in the near activation state or below the near activation state), the method 1000 loops back to block 1002 to continue monitoring user input. If the user input indicates activation of cutting, the method 1000 advances to block 1010.

In block 1010, the control element causes an indication that cutting has started, which is simultaneous (or otherwise close in time) with activation of the cutting energy. For example, the surgical instrument 102 may activate ultrasound or RF energy to begin the cutting operation (i.e., transection). The indication that cutting has begun may be different from the indication that cutting is about to begin. For example, the haptic feedback pattern for cutting may be different in amplitude, frequency, or other parameters from the haptic feedback pattern that indicates that cutting is about to begin. Accordingly, in a sequential coagulation then cut process, the system 100 may provide feedback that allows the user to confirm advancement to the next stage (e.g., from coagulation to cutting). After indicating cutting has started, the method 1000 loops back to block 1002 to continue monitoring user input.

Referring now to FIG. 11, a method 1100 for controlling an energy-based surgical instrument 102 is shown. The method 1100 may be executed by the controller 702, the generator 106, and/or one or more other microcontrollers or other control elements of the system 100. The method 1100 begins in block 1102, in which the control element measures tension applied to tissue by the surgical instrument 102. For example, towards the end of a transection when using an ultrasonic device 102, users sometimes have a tendency to apply tension on the tissue being transected by pulling up on the distal end of the device 102. This extra tension that users apply on the tissue can cause the tissue to transect faster and sooner than if no extra tension were applied.

In block 1104, the control element generates haptic feedback based on the measured tension. As described above, the haptic feedback is generated with the haptic device 810, for example, a vibration motor, a speaker, a haptic transducer, or other haptic feedback component included in the handle of the surgical instrument 102. In some embodiments, in block 1106 the control element may generate the haptic feedback in order to notify the user of an amount of tension being applied. For example, the measured tension may be communicated to the user, which may allow the user to not apply extra tension during a transection, as the extra tension may cause hemostasis issues. Continuing that example, a haptic algorithm may apply pulses in varying intensity indicative of the applied tension, which may remind the users to not apply extra tension and otherwise improve user technique. The haptic algorithm can also be used to notify the user of the amount of tension the user is applying. Longer, slower haptics could indicate an appropriate amount of tissue tension, whereas a more frequent pulse could indicate too much tension is being applied.

In some embodiments, in block 1108 the control element may generate the haptic feedback to indicate whether the current tension is conducive to hemostasis, for example when using the ultrasonic blade 130 to score or transect tissue with open jaws. Varying haptic feedback to the user could also be used when an ultrasonic blade 130 is being used to score or transect tissue with open jaws. The haptic feedback could be directly related to the amount of tension being applied on the blade (blade deflection) and the feedback could decrease in frequency or intensity when the user is scoring tissue at a pace or tension conducive to delivering hemostasis to the tissue. The haptic feedback could increase frequency or intensity to indicate to the user to apply less tension at the blade and move the blade slower to obtain hemostasis. After generating the haptic feedback, the method 1100 loops back to block 1102 to continue monitoring tension and generating feedback.

The following is a non-exhaustive list of embodiments which may or may not be claimed:
1. A method for controlling an energy-based surgical instrument, the method comprising:
   determining, by a control element, an instrument state of the energy-based surgical instrument;
   determining, by the control element, a feedback indication based on the instrument state, wherein the feedback indication is indicative of the instrument state and is interpretable by a user of the surgical instrument; and
   outputting, by the control element, the feedback indication with a feedback device.
2. The method of embodiment 1, wherein determining the instrument state comprises determining an activation mode for the surgical instrument, wherein the activation mode comprises automatic mode or latch mode.
3. The method of embodiment 1, wherein determining the instrument state comprises determining an energy modality for the surgical instrument, wherein the energy modality comprises ultrasound or radio frequency (RF).
4. The method of embodiment 1, wherein determining the instrument state comprises determining a requested activation status based on a user input.
5. The method of embodiment 1, wherein determining the instrument state comprises determining an operational status of the surgical instrument based on instrument data.
6. The method of embodiment 5, wherein the operational status comprises energy deactivated or energy activated.
7. The method of embodiment 1, wherein outputting the feedback indication comprises generating haptic feedback with a haptic device coupled to a handpiece of the surgical instrument.
8. The method of embodiment 1, wherein:
   determining the instrument state comprises determining an activation mode for the surgical instrument based on a mode selector switch of the surgical instrument, wherein the activation mode comprises automatic mode or latch mode;
   determining the feedback indication comprises determining a first haptic pattern when the activation mode comprises the automatic mode and a second haptic pattern when the activation mode comprises the latch mode; and
   outputting the feedback indication comprises outputting the first haptic pattern or the second haptic pattern with a haptic device coupled to the surgical instrument.
9. The method of embodiment 1, wherein:
   determining the instrument state comprises determining a requested activation status based on an actuation state of a user input, wherein the user input is operatable over a range of actuation from not actuated to fully actuated;
   determining the feedback indication comprises determining a first haptic pattern when the actuation state of the user input reaches a first position between not actuated and fully actuated; and determining a second haptic pattern when the actuation state of the user input reaches fully actuated; and
   outputting the feedback indication comprises outputting the first haptic pattern or the second haptic pattern with a haptic device coupled to the surgical instrument.
10. The method of embodiment 9, wherein the user input comprises a primary trigger of the surgical instrument.
11. The method of embodiment 9, further comprising activating, by the control element, energy delivery with the surgical instrument simultaneously with outputting the second haptic pattern when the actuation state of the user input reaches fully actuated.
12. The method of embodiment 9, wherein outputting the feedback indication comprises outputting the second haptic pattern sequentially after outputting the first haptic pattern.
13. The method of embodiment 1, wherein:
   determining the instrument state comprises measuring tension on an end effector of the surgical instrument while applying ultrasound energy with the end effector;
   determining the feedback indication comprises determining a first haptic pattern based on the measured tension; and
   outputting the feedback indication comprises outputting the first haptic pattern with a haptic device coupled to the surgical instrument.
14. The method of embodiment 13, wherein determining the first haptic pattern based on the measured tension comprises increasing a frequency of haptic feedback pulses as an amount of the measured tension increases.
15. The method of embodiment 13, wherein determining the first haptic pattern based on the measured tension comprises decreasing a frequency of haptic feedback pulses as an amount of the measured tension nears a predetermined amount of tension, wherein the predetermined amount of tension is conducive for hemostasis.
16. A system for controlling an energy-based surgical instrument, the system comprising:
   an energy-based surgical instrument comprising an end effector;
   a feedback device coupled to the surgical instrument; and
   a control element configured to (i) determine an instrument state of the energy-based surgical instrument, (ii) determine a feedback indication based on the instrument state, wherein the feedback indication is indicative of the instrument state and is interpretable by a user of the surgical instrument, and (iii) output the feedback indication with the feedback device.
17. The system of embodiment 16, wherein the energy-based surgical instrument comprises a haptic device coupled to a handpiece of the surgical instrument, and wherein to output the feedback indication comprises to generate haptic feedback with the haptic device.
18. The system of embodiment 16, wherein:
   to determine the instrument state comprises to determine an activation mode for the surgical instrument based on a mode selector switch of the surgical instrument, wherein the activation mode comprises automatic mode or latch mode;
   to determine the feedback indication comprises to determine a first haptic pattern when the activation mode comprises the automatic mode and a second haptic pattern when the activation mode comprises the latch mode; and
   to output the feedback indication comprises to output the first haptic pattern or the second haptic pattern with a haptic device coupled to
   the surgical instrument.
19. The system of embodiment 16, wherein:
   to determine the instrument state comprises to determine a requested activation status based on an actuation state of a user input, wherein the user input is operatable over a range of actuation from not actuated to fully actuated;
   to determine the feedback indication comprises to determine a first haptic pattern when the actuation state of the user input reaches a first position between not actuated and fully actuated; and to determine a second haptic pattern when the actuation state of the user input reaches fully actuated; and
   to output the feedback indication comprises to output the first haptic pattern or the second haptic pattern with a haptic device coupled to
   the surgical instrument.
20. The system of embodiment 16, wherein:
   to determine the instrument state comprises to measure tension on an end effector of the surgical instrument during application of ultrasound energy with the end effector;
   to determine the feedback indication comprises to determine a first haptic pattern based on the measured tension; and
   to output the feedback indication comprises to output the first haptic pattern with a haptic device coupled to the surgical instrument.

While the disclosure has been illustrated and described in detail in the drawings and foregoing description, such an illustration and description is to be considered as illustrative and not restrictive in character, it being understood that only illustrative embodiments have been shown and described and that all changes and modifications that come within the spirit of the disclosure are desired to be protected.

There are a plurality of advantages of the present disclosure arising from the various features of the methods, apparatuses, and systems described herein. It will be noted that alternative embodiments of the methods, apparatuses, and systems of the present disclosure may not include all of the features described yet still benefit from at least some of the advantages of such features. Those of ordinary skill in the art may readily devise their own implementations of the methods, apparatuses, and systems that incorporate one or more of the features of the present invention and fall within the spirit and scope of the present disclosure as defined by the appended claims.

## Claims

1. A system for controlling an energy-based surgical instrument, the system comprising:
an energy-based surgical instrument comprising an end effector;
a feedback device coupled to the surgical instrument; and
a control element configured to (i) determine an instrument state of the energy-based surgical instrument, (ii) determine a feedback indication based on the instrument state, wherein the feedback indication is indicative of the instrument state and is interpretable by a user of the surgical instrument, and (iii) output the feedback indication with the feedback device.

2. The system of claim 1, wherein the energy-based surgical instrument comprises a haptic device coupled to a handpiece of the surgical instrument, and wherein to output the feedback indication comprises to generate haptic feedback with the haptic device.

3. The system of claim 1 or claim 2, wherein:
to determine the instrument state comprises to determine an activation mode for the surgical instrument based on a mode selector switch of the surgical instrument, wherein the activation mode comprises automatic mode or latch mode;
to determine the feedback indication comprises to determine a first haptic pattern when the activation mode comprises the automatic mode and a second haptic pattern when the activation mode comprises the latch mode; and
to output the feedback indication comprises to output the first haptic pattern or the second haptic pattern with a haptic device coupled to the surgical instrument.

4. The system of any preceding claim, wherein:
to determine the instrument state comprises to determine a requested activation status based on an actuation state of a user input, wherein the user input is operatable over a range of actuation from not actuated to fully actuated;
to determine the feedback indication comprises to determine a first haptic pattern when the actuation state of the user input reaches a first position between not actuated and fully actuated; and to determine a second haptic pattern when the actuation state of the user input reaches fully actuated; and
to output the feedback indication comprises to output the first haptic pattern or the second haptic pattern with a haptic device coupled to the surgical instrument.

5. The system of any preceding claim, wherein:
to determine the instrument state comprises to measure tension on an end effector of the surgical instrument during application of ultrasound energy with the end effector;
to determine the feedback indication comprises to determine a first haptic pattern based on the measured tension; and
to output the feedback indication comprises to output the first haptic pattern with a haptic device coupled to the surgical instrument.

6. A computer-implemented method for controlling an energy-based surgical instrument, the method comprising:
determining, by a control element, an instrument state of the energy-based surgical instrument;
determining, by the control element, a feedback indication based on the instrument state, wherein the feedback indication is indicative of the instrument state and is interpretable by a user of the surgical instrument; and
outputting, by the control element, the feedback indication with a feedback device.

7. The method of claim 6, wherein determining the instrument state comprises determining one or more of:
(i) an activation mode for the surgical instrument, wherein the activation mode comprises automatic mode or latch mode;
(ii) an energy modality for the surgical instrument, wherein the energy modality comprises ultrasound or radio frequency (RF);
(iii) a requested activation status based on a user input;
(iii) an operational status of the surgical instrument based on instrument data, optionally wherein the operational status comprises energy deactivated or energy activated.

8. The method of claim 6 or claim 7, wherein outputting the feedback indication comprises generating haptic feedback with a haptic device coupled to a handpiece of the surgical instrument.

9. The method of any one of claims 6 to 8, wherein:
determining the instrument state comprises determining an activation mode for the surgical instrument based on a mode selector switch of the surgical instrument, wherein the activation mode comprises automatic mode or latch mode;
determining the feedback indication comprises determining a first haptic pattern when the activation mode comprises the automatic mode and a second haptic pattern when the activation mode comprises the latch mode; and
outputting the feedback indication comprises outputting the first haptic pattern or the second haptic pattern with a haptic device coupled to the surgical instrument.

10. The method of any one of claims 6 to 9, wherein:
determining the instrument state comprises determining a requested activation status based on an actuation state of a user input, wherein the user input is operatable over a range of actuation from not actuated to fully actuated;
determining the feedback indication comprises determining a first haptic pattern when the actuation state of the user input reaches a first position between not actuated and fully actuated; and determining a second haptic pattern when the actuation state of the user input reaches fully actuated; and
outputting the feedback indication comprises outputting the first haptic pattern or the second haptic pattern with a haptic device coupled to the surgical instrument.

11. The method of claim 10, wherein the user input comprises a primary trigger of the surgical instrument.

12. The method of claim 10 or claim 11, further comprising activating, by the control element, energy delivery with the surgical instrument simultaneously with outputting the second haptic pattern when the actuation state of the user input reaches fully actuated.

13. The method of any one of claims 10 to 12, wherein outputting the feedback indication comprises outputting the second haptic pattern sequentially after outputting the first haptic pattern.

14. The method of any one of claims 6 to 13, wherein:
determining the instrument state comprises measuring tension on an end effector of the surgical instrument while applying ultrasound energy with the end effector;
determining the feedback indication comprises determining a first haptic pattern based on the measured tension; and
outputting the feedback indication comprises outputting the first haptic pattern with a haptic device coupled to the surgical instrument.

15. The method of claim 14, wherein determining the first haptic pattern based on the measured tension comprises one or both of:
(i) increasing a frequency of haptic feedback pulses as an amount of the measured tension increases; and
(ii) decreasing a frequency of haptic feedback pulses as an amount of the measured tension nears a predetermined amount of tension, wherein the predetermined amount of tension is conducive for hemostasis.
